(19) Europäisches Patentamt
European Patent Office
Office européen des brevets

(11) **EP 2 988 782 B1**

(12) **EUROPEAN PATENT SPECIFICATION**

(45) Date of publication and mention of the grant of the patent:
**22.05.2019 Bulletin 2019/21**

(21) Application number: **14731011.4**

(22) Date of filing: **22.04.2014**

(51) Int Cl.:
*A61K 9/20* *(2006.01)*        *A61K 47/36* *(2006.01)*
*A61K 9/28* *(2006.01)*        *A61K 31/05* *(2006.01)*
*A61K 31/07* *(2006.01)*        *A61K 31/10* *(2006.01)*
*A61K 31/122* *(2006.01)*        *A61K 31/198* *(2006.01)*
*A61K 31/355* *(2006.01)*        *A61K 31/525* *(2006.01)*

(86) International application number:
**PCT/IB2014/060892**

(87) International publication number:
**WO 2014/174430 (30.10.2014 Gazette 2014/44)**

(54) **DOSAGE FORMS FOR ORAL ADMINISTRATION OF ACTIVE SUBSTANCES**

DARREICHUNGSFORMEN ZUR ORALEN VERABREICHUNG VON WIRKSTOFFEN

FORMES PHARMACEUTIQUES POUR L'ADMINISTRATION ORALE DE SUBSTANCES ACTIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.04.2013 IT MI20130682**

(43) Date of publication of application:
**02.03.2016 Bulletin 2016/09**

(73) Proprietor: **Giuliani S.p.A.**
**20129 Milano (IT)**

(72) Inventors:
• **GIULIANI, Giammaria**
**I-20123 Milano (IT)**
• **BENEDUSI, Anna**
**I-20124 Milano (IT)**
• **MASCOLO, Antonio**
**I-20126 Milano (IT)**
• **LIMITONE, Antonio**
**I-20132 Milano (IT)**

(74) Representative: **Coppo, Alessandro et al**
**Notarbartolo & Gervasi S.P.A.**
**Corso di Porta Vittoria, 9**
**20122 Milano (IT)**

(56) References cited:
EP-A1- 1 666 518        WO-A2-2006/087392
US-A1- 2010 098 794

**Description**

FIELD OF THE INVENTION

The present invention relates to dosage forms for oral administration of active substances.

The present invention originates in the field of solid systems in the form of a tablet or capsule suitable for oral administration, and for the controlled release of active ingredients.

STATE OF THE ART

The systemic administration of a substance or active ingredient (AP) requires that a substance to be administered be incorporated or conveyed in a dosage form.

The route of oral administration is a more practical and recurrent route of administration, because it allows an active substance to be ingested and assimilated along the gastrointestinal tract. This form of administration is an effective and convenient way to obtain blood levels of the active ingredient in a desired range and relatively short time.

The maintenance of blood levels of the active ingredient in time, below the toxic threshold and above the concentration in which they are ineffective, suggests that strategies be adopted to maintain the blood concentration of the active ingredient within the level of therapeutic efficacy, in a time span as long as possible.

This purpose can be pursued through different methods.

One of these consists in the administration of multiple doses throughout the day. This approach involves the occurrence of disadvantages, since the repeated administration of the active ingredient, typically a drug, at regular intervals, in addition to having a low compliance may determine the occurrence of unsuitable blood profiles.

To overcome this drawback, systems for the controlled release of the active ingredients have been developed over time.

The main advantages of these systems are as follows:

- increased compliance of the patient to the drug, due to a reduction in the frequency of drug intake over time (e.g. once a day).
- increase of the tolerability of the drug with reduced side effects.
- greater effectiveness: several clinical studies have shown that for many drugs the patient responds best to minimum doses for long periods of time.

One of the main goals that we aim to achieve by adopting these techniques is to regulate the delivery of the active ingredient to be conveyed in the body, by controlling its release rate over time or space. Plasma levels of the active ingredient can thus be obtained within the range of the therapeutic effectiveness, reducing the frequency and often also the amount of drug to be administered daily, and reducing the occurrence of side effects and the related risks.

Some specific forms of modified release of active ingredientss are those of the enteric type, which are used to avoid or considerably reduce the release of the active ingredient within the stomach.

These dosage forms are designed to resist the acid pH of the stomach gastric juices and to release the active ingredient in an environment in which higher pH values are present, typically between 5.5 and 6.5, as occurs in the small intestine and colon, respectively.

It is also known to use hydrophilic polymers such as, for example, cellulose ethers, in the formulation of preparations for oral administration.

The variables that affect the release of active ingredientss present in these systems are many and are linked to the choice of the polymer matrix type, to the many chemical and physical characteristics of the active ingredientss, but also of the excipients present in the formulation.

Other factors such as the permeability (pore permeability), shape and size of the tablet, affect the release kinetics of the active ingredient contained in the formulation as well.

To modulate the release of an active ingredient, a procedure can be used according to which poorly water soluble active ingredientss are put in contact with very soluble substances, in order to facilitate the solubilization or dispersion of the actives in the aqueous medium. Substances commonly used for these purposes are alcohols, polyalcohols, amphiphilic substances such as surfactants or mixtures of substances which, in contact with water, produce effervescence such as a mixture of acids with carbonates and bicarbonates.

Also known is the possibility of placing hydrophobic substances in contact with ingredients for which the affinity with water should be reduced. WO2006/087392 discloses compositions of ultra-low molecular weight hyaluronic acid oligomers which are formulated in fluid or solid form.

The use of specific technologies and pharmaceutical equipment specifications allows for changes to the release profile of active substances. However, the use of complementary formulation technologies causes an increase in the complexity of the formulation study with a consequent increase of costs and development time. One of the objects of the present invention is to provide a solid dosage form for the controlled release of one or more active ingredients, which is simple to implement and does not entail high realization costs.

SUMMARY OF THE INVENTION

In a first aspect, the object of the present invention is a solid system in the form of a tablet or capsule for the modified release and oral administration of one or more active ingredients, comprising a polymeric matrix comprising an ionic polymer which is hyaluronic acid in the form of free acid with a weighted-average molecular weight ranging from 100 to 100,000 Daltons (Da) measured with SEC-MALLS technique, and a non-ionic polymer which jellifies in contact with a liquid, and an active ingredient dispersed in a polymeric matrix.

The Applicant has unexpectedly found that the use of a polymeric matrix which contains an ionic polymer, namely hyaluronic acid having a selected MW ranging from 100 to 100,000 Da, preferably from 500 to 50,000 Daltons, and a non-ionic polymer which jellifies in contact with water, makes it possible to modulate effectively the release of active ingredients contained or dispersed in the same polymer matrix.

The hyaluronic acid used in the present context is in the form of free acid, that is in non-salified form.

In one embodiment of the invention, said matrix modified release system involves the use of a plurality of systems such as microgranules and microcapsules containing one or more active ingredients, conveniently chosen through an innovative calculation method.

Typically, the solid system disclosed herein is a dynamic system regulated by a series of successive events such as the hydration of the polymer, gel formation, swelling and solubilization of the polymer; the latter being an event that determines the matrix erosion.

At the same time, active and non-active soluble ingredients, present in the polymeric matrix, become soaked by the water that permeates through the surface layers of the composition. Once the ingredients are dissolved, they may diffuse through the gel layer while the insoluble components remain in the matrix until hydration and dissolution of the polymer and the progressive erosion of the outermost layers of the matrix in gastric fluid have occurred.

The Applicant has found that when the polymer matrix contains a non-ionic polymer and the hyaluronic acid is absent, modulating the release of active ingredient dispersed in the polymer matrix or contained in the microgranules becomes difficult.

Moreover, the Applicant has found that when the hyaluronic acid is absent from the polymer matrix, small variations in the amount of the non-ionic polymer present in the same matrix, e.g. 1-5% on the weight of the composition, cause a considerable variation of the dissolution speed of the active ingredient in an aqueous environment, e.g. 5-25%.

In one embodiment of the invention, the polymeric matrix contains hyaluronic acid with molecular weight ranging from 500 to 50,000 Da.

Typically, the solid system for modified release and/or administration of one or more active ingredients is for pharmaceutical use.

In some embodiments, the polymeric matrix contains one or more non-ionic polymers, whose times of hydration and/or gel formation are independent of the pH and properly selected from cellulose derivatives, ethylene oxide polyether and mixtures thereof.

In some embodiments, microgranules and microcapsules containing one or more active ingredients may be obtained by treating functional substances or active ingredients with appropriate adjuvants, which may be selected by an innovative calculation method, described below, that can reduce the formulation development time of solid dosage forms containing one or more active ingredients for which the delivery is to be modified.

This method is particularly effective and advantageous in dosage forms containing more active ingredients, such as multivitamin products, those containing minerals and trace elements, plant extracts, products containing probiotics.

Consequently, in accordance with some embodiments of the invention, the active ingredient present in microspheres or microcapsules with a hydrophilicity/lipophilicity index ($I_{p.a.}$) is combined with one or more adjuvants, typically amphiphilic, having an overall hydrophilicity/lipophilicity index ($I_c$):

-   greater than $I_{p.a.}$ to increase the release of AP; or
-   lesser than $I_{p.a.}$ to decrease the release of AP;

wherein

$$l.p.a. = \frac{9000\,e^x - 9000\,e^{-x}}{450e^x - 450e^{-x} + 10} \cdot \frac{x^{30}}{x^{30} + 0.01}$$

and

$$Ic = \frac{MWi}{MW} \cdot 20$$

Where X is equal to the solubility S (mg/liter)/dose (mg/dose).

The solubility S of the AP is expressed in mg per 1000 ml of water at the temperature of 20°C-25°C, and where MWi is the molecular weight of the hydrophilic portion of the adjuvant and MW is the molecular weight of the entire adjuvant.

Application of said method can be advantageous, for example, when the characteristics of the active ingredients are very different from one another, so that an approach based on a plurality of combined strategies allows a suitable formulation flexibility to be obtained, with consequent reducing the development time and cost.

One of the characteristic of such a solid system is the wide versatility, as it is applicable to active ingredients with very different chemical characteristics and does not require excessively complex equipment, different from those generally available in the workshops engaged in the production of solid dosage forms, or at producers of raw materials for pharmaceutical or other industry.

An object of this solid system is also to improve the bioavailability of active ingredients.

DETAILED DESCRIPTION OF THE INVENTION

According to a first aspect, the present invention provides a solid system in the form of a tablet or capsule for the modified release and oral administration of one or more active ingredients as defined in the appended claim 1.

For the purposes of the present invention, the Applicant has surprisingly found that hyaluronic acid and sodium hyaluronate, or other salts of hyaluronic acid, are not equivalent, in particular for the purposes of obtaining the modulation of the release of active ingredient from the polymer matrix.

The hyaluronic acid used herein is a glycosaminoglycan characterized by the repetition of a disaccharide unit consisting of glucuronic acid bound by glycosidic bond $\beta1{\rightarrow}4$ and $\beta1{\rightarrow}3$ to N-acetylglucosamine.

At a pH higher than that of the stomach, strongly acid and generally varying from 1 to 3, the carboxylic groups of glucuronic units are ionized, a condition that gives the molecule a high polarity and accordingly a high water solubility. Thanks to this property, the hyaluronic can establish a number of intramolecular and intermolecular interactions with other polymer or water molecules, thus reaching a high hydration degree.

In some embodiments, the hyaluronic acid has a molecular weight ranging from 500 to 50,000 Da, and preferably has a MW ranging from 800 to 1500 Da. Typically, the molecular weight of the hyaluronic acid of the release solid system is determined with SEC-MALLS technique, or multi-angle laser light scattering - size exclusion chromatography.

According to some embodiments, the above polymeric matrix comprises a polymeric mixture of a non-ionic polymer which, in contact with water, swells and forms a gel, and a hyaluronic acid based ionic polymer having MW as previously described.

Suitable non-ionic polymers are polymers which, in contact with the water, gellify. Particularly suitable non-ionic polymers are those for which hydration and gel formation time are not affected by the environmental pH.

Suitable non-ionic polymers are cellulose-based or cellulose derivatives, such as cellulose ethers or esters.

Suitable cellulose esters include organic esters such as cellulose acetate, cellulose triacetate, cellulose propionate, cellulose acetate propionate (CAP), cellulose acetate butyrate (CAB), or inorganic esters such as nitrocellulose, cellulose sulfate and mixtures of organic and/or inorganic esters.

Suitable cellulose ethers include i) alkyl ethers including methylcellulose, ethylcellulose, ethylmethyl cellulose; ii) hydroxyalkyl ethers such as hydroxyethyl cellulose, hydroxypropyl cellulose (HPC), hydroxyethyl methylcellulose, hydroxypropyl methylcellulose (HPMC), ethylhydroxy ethylcellulose, or iii) carboxyalkyl cellulose ethers such as carboxymethyl cellulose (CMC).

Among the cellulose based non-ionic polymers also falls hemicellulose.

Further suitable non-ionic polymers include polyethylene glycols or PEG, which may have molecular weight between 300 and 10,000,000 g/mol, typically from 100 to 50,000 g/mol, in particular from 1000 to 20,000 g/mol.

In the context of the present disclosure, the terms polyethylene glycol (PEG), polyethylene oxide (PEO) or polyoxyethylene (POE) are interchangeable and refer to a polyether prepared by the polymerization of ethylene oxide.

For the purposes of the present invention, with microgranules we mean solid preparations consisting of aggregates sufficiently resistant to manipulation in which a clear distinction between internal and external structure cannot be defined. In this solid system, the active ingredient is dissolved or finely dispersed.

For the purposes of the present disclosure, with microcapsules we mean vesicular systems characterized by a central core and a continuous coating or outer membrane.

Suitable adjuvants or useful excipients for the preparation of said microgranules can be chosen among the following substances: salts of fatty acids, mono and diglycerides of fatty acids, esters of fatty acids with glycerol and polyglycerol and lecithins.

For the purposes of the present disclosure, lecithins can be used which are obtained from vegetable raw materials (e.g. *Glycine max L., Helianthus annuus, Brassica carinata* etc.) and animal substances (e.g. egg yolk) and specific phospholipids with high purity degree, as well as lecithins and synthetic phospholipids.

For the purposes of the present disclosure, the use of soy lecithin (*Glycine max* L.) is preferred, that is the concentrated phospholipid obtained through degumming of the oil fraction extracted from the seeds of soybean. The resulting product is mainly made up of sn-glycerol-3-phosphate glycerophospholipids, such as phosphatidylcholine, phosphatidylinositol, phosphatidylethanolamine, phosphatidic acid and so on.

The specific lecithins or phospholipids may also be dissolved or diluted in ethyl alcohol, which allows a more durable preservation thereof, and used as such for the preparation of a granulate or a semisolid mixture.

Other suitable adjuvants include polyoxyethylene stearate 40 (Ic approximately 17.5); polysorbate 20 (Ic approximately 16.7), polysorbate 80 (Ic approximately 15), polysorbate 40 (Ic approximately 15.6), polysorbate 65 (Ic approximately 10.5), sucroesteri such as (sucrose laurate, sucrose stearate), esters of sorbitan (e.g. sorbitan monostearate Ic about 4.7; sorbitan monolaurate Ic about 8.6; sorbitan monooleate Ic about 4.3, sorbitan monopalmitate Ic about 4.7).

For the purposes of the present disclosure, substances or active ingredients contained in microgranules comprise substances or active ingredients suitable for systemic administration, whether they are equipped with therapeutic, nutritional, dietetic or beneficial activity for the organism.

Suitable active ingredients comprise water-soluble and/or fat-soluble vitamins, amino acids, minerals, pharmacologically active ingredients; mixtures of vegetable ingredients such as plant extracts and/or phytochemicals; live microorganisms provided with dietary action such as probiotics, which, when ingested, have one or more functional effects or benefits on the health of the host.

For example, suitable active ingredients include phytocompounds such as ellagic acids, anthocyanins, catechins, sulfur compounds, phytosterols or plant sterols, flavonols, isoflavones of soybeans and other legumes, lignans, terpenes, l'oleuropein and hydroxytyrosol, resveratrol, saponins, tannins.

Suitable active ingredients contained in the solid system disclosed herein may be plant extracts, such as maritime pine bark extract Pycnogenol® (Horphag Research), epigallocatechin gallate (Teavigo - DSM Nutritional Products Ltd), extract of Ajuga reptans (labiatae) for example, standardized to 50% of phenylpropanoids expressed as teupolioside (IRB - Istituto di Ricerche Biotecnologiche).

The solid system may contain one or more active ingredients in a biologically or pharmaceutically acceptable quantity.

In accordance with some embodiments, the solid system disclosed herein further comprises an outer enteric coating. The thickness of the outer coating is that typical of enteric formulations or preparations used in the pharmaceutical, dietary or nutritional field.

The gastric resistance characteristics are generally imparted to the composition of the invention using polymeric substances of various nature, such as cellulose derivatives, for example cellulose acetate phthalate, cellulose acetate propionate, acrylic and methacrylic acid derivatives.

Also suitable are polymers of natural origin, such as shellac, modified starches (e.g. Amprac) and alginates.

The Amprac, a modified corn starch, is a polymer insoluble at temperatures below 50°C. Its use requires high process temperatures, which is not always possible or convenient to use.

Shellac is a natural polymer, obtained by refining the secretions of an insect of the family of hemiptera (*Kerria lacca*) and comprises a mixture of polyesters including aleuretic and shelloic acid.

A suitable alginate is the sodium salt of alginic acid, a polyuronic acid present in different species of marine seaweed. Alginic acid is a linear polymer consisting of mannuronic acid residues bound together in such a manner as to leave a free carboxyl in each unit. The length of the chain depends on the type of marine seaweed and the state of maturation of the same.

In certain embodiments, lipophilic substances, such as stearic acid, are added to the alginic acid, to increase the hydrophobicity of the polymer.

In some embodiments, this enteric outer coating comprises alginic acid or salts thereof, with alkali metals, and/or shellac.

The solid system disclosed herein may further comprise excipients conventional in the pharmaceutical or dietary, nutritional products such as diluents, lubricants, anti-caking agents, humectants, aggregating, disintegrants, and mixtures thereof. The solid system of the invention is in the form of tablet or capsules for the modified release and oral administration.

According to another aspect, a solid system in the form of a tablet or capsule comprising a polymeric matrix based on hyaluronic acid with molecular weight ranging from 100 to 100,000 Daltons for use in modulating the release of one or more active ingredients from a composition is disclosed herein.

The polymeric matrix further contains a non-ionic polymer of the type previously described.

Not claimed are certain embodiments, in which the solid system for modified release of active ingredients is a composition typically for pharmaceutical use, or a medical device comprising one or more physiologically or pharmaceutically acceptable excipients.

In some embodiments the active ingredients present in the polymer matrix are contained in microparticles or microcapsules.

In certain embodiments, hyaluronic acid has molecular weight ranging from 500 to 50,000 Daltons.

A solid system for the use according to claim 9 is also provided.

Typically, polymers, such as the hyaluronic acid and/or PEG, possibly present in the polymer matrix of the disclosed release solid system, have a molecular weight that is a weighted-average molecular weight (Mw), obtained as previously described.

The following examples are provided.

EXAMPLE 1

Examples using hyaluronic acid in the form of free acid are according to the invention.

Description of a solid system for the release of actives with mixed polymer matrix formed by a non-ionic polymer and a matrix based on ionic polymer of hyaluronic acid.

Four types of polymers were found, which are listed in the following table.

| |
|---|
| Hyaluronic acid, sodium salt m.w. $10^3$ kilo daltons kDa "high molecular weight" |
| Hyaluronic acid, sodium salt m.w. 250 - 400 (kDa). "low molecular weight" |
| Hyaluronic acid <1000 Da "low molecular weight" |
| Hyaluronic acid <100,000 Da "low molecular weight" |

A system for the release of actives in the form of tablets was therefore produced using a tabletting machine (8-punch rotary tabletting machine Minipress- - Officine Meccaniche F.Ili Ronchi, Cinisello Balsamo - MI), and a set of oval punches 16.4 x 9.3 mm, curved, dual-beam, anonymous.

Riboflavin was used as a water-soluble active ingredient because, as it is colorful, the release and the evolution of the tablet can be verified by visual observation within a dissolution assay of tablets, performed according to the method reported in USP 35.

Tablets containing the riboflavin active ingredient were produced in combination with a modifier of the release kinetics according to the matrix mechanism, such as hydroxypropyl methylcellulose alone and in association with hyaluronic acid with low molecular weight and its salt with low and high molecular weight.

The composition of the tested formulations are shown in the following Table.

| Ingredient of the system | Test 13 | Test 14 | Test 15 | Test 17 | Test 18 | Test 19 |
|---|---|---|---|---|---|---|
| | % | % | % | % | % | % |
| Dicalcium phosphate | 50.79 | 51.79 | 52.79 | 50.79 | 50.79 | 50.79 |
| Microcrystalline cellulose | 40.00 | 40.00 | 40.00 | 40.00 | 40.00 | 40.00 |
| Hydroxypropyl methylcellulose | 7.60 | 6.60 | 5.60 | 6.60 | 6.60 | 6.60 |

(continued)

| Ingredient of the system | Test 13 | Test 14 | Test 15 | Test 17 | Test 18 | Test 19 |
|---|---|---|---|---|---|---|
| | % | % | % | % | % | % |
| Hyaluronic acid, sodium salt MW 1*10 exp 6 Da | | | | 1.00 | | |
| Hyaluronic acid, sodium salt MW 250 - 400 exp 3 Da | | | | | | 1.00 |
| Hyaluronic acid MW 1*10 exp 3 Da | | | | | 1.00 | |
| Magnesium stearate | 0.90 | 0.90 | 0.90 | 0.90 | 0.90 | 0.90 |
| Riboflavin | 0.21 | 0.21 | 0.21 | 0.21 | 0.21 | 0.21 |
| Silicon dioxide | 0.50 | 0.50 | 0.5 | 0.5 | 0.5 | 0.5 |
| | 100 | 100 | 100 | 100 | 100 | 100 |

The three formulas containing only HPMC were intended to confirm the on-off defined behavior for this raw material, that produces, even with small variations of the polymer, significant differences in the release kinetics.

The greatest risk in the case of supplements, where the active ingredients are vitamins and trace elements, which are used within the indication given by the national RDA or LARN, is either not getting a real modulation of the active ingredient or, conversely, not getting at all the release of the same.

In the case of an active pharmaceutical principle, the modulation effect is of relevance in the context of improving the therapeutic efficacy.

The other tablets were made with combinations of HPMC (6.6%) and sodium hyaluronate (1%) with low and high molecular weight, together with tablets including HPMC (6.6%) - hyaluronic acid (1%).

The tablets produced were compared with regard to, thickness, hardness and friability. The batches were comparable to each other with respect to these three parameters. As regards the release profile of the substance over time, as observed in the course of the dissolution test made according to the USP method, it has been observed that tablets made with combinations of HPMC (6.6%) and sodium hyaluronate (1%) with high molecular weight, showed a behavior comparable to those containing HPMC alone (7.6%). Similarly, tablets made with HPMC (6.6%) and sodium hyaluronate (1%) with low molecular weight have been shown to disaggregate already after six hours from the beginning of the assay, pointing out a disaggregating behavior of sodium hyaluronate with the lowest polymerization degree. The tablets with only HPMC 6.6% were fully disaggregated already after 6 hours, whereas the tablets containing HPMC 7.6% were still well-structured after 8 hours of the assay.

It is unexpectedly observed that tablets of a single active ingredient in combination with modifiers of the release kinetics with a polymeric matrix containing HPMC (6.6%) and hyaluronic acid (1%) with low molecular weight showed a dissolution profile more linear in time, accompanied by a total breakdown of the nucleus, that occurred between the seventh hour and the eighth hour of the test; only in certain cases the presence was found of a soft, impalpable, hydrated nucleus, almost discharged of active ingredient.

The matrix release system involves the passage of soluble substances molecules through the swollen gel matrix, while for the fat-soluble molecules the progressive erosion of said gel allows the passage of the substance in the medium. In the case of fat-soluble substances, therefore, the uncomplete breakdown during the dissolution assay is equivalent to showing that these substances are not available for their assimilation. The use of riboflavin as a test substance for the water-soluble actives is due to its color, the presence of a disaggregated orange nucleus at the end of the essay is equivalent to proving that the riboflavin has not been released into the liquid medium.

The theses listed above were then repeated using the beadlet of zeaxanthin (zeaxanthin 5%), also of a vibrant orange color.

| Ingredient | Test 23 | Test 24 | Test 25 |
|---|---|---|---|
| | % | % | % |
| Dicalcium phosphate | 45.61 | 45.61 | 45.61 |
| Microcrystalline cellulose | 40.00 | 40.00 | 40.00 |
| Hydroxypropyl methylcellulose | 6.60 | 6.60 | 6.60 |
| Hyaluronic acid, sodium salt MW 1*10 exp 6 Da | 1.00 | | |
| Hyaluronic acid, sodium salt MW 250 - 400 exp 3 Da | | | 1.00 |

(continued)

| Ingredient | Test 23 | Test 24 | Test 25 |
|---|---|---|---|
| | % | % | % |
| Hyaluronic acid MW 1*10 exp 3 Da | | 1.00 | |
| Magnesium stearate | 0.90 | 0.90 | 0.90 |
| Zeaxanthin vegetarian beadlets 5% | 5.39 | 5.39 | 5.39 |
| Silicon dioxide | 0.5 | 0.5 | 0.5 |
| | 100 | 100 | 100 |

The observed results confirmed what was seen for riboflavin.

The presence of hyaluronic acid in the polymer matrix is significant to the realization of modified-release tablets.

It was therefore decided to repeat the assay and to follow analytically by HPLC-UV, the delivery of the active riboflavin at various stages (after 2 hours of acid pH, 6 and 8 hours).

However, this investigation has provided results not consistent with each other due to the different solubility of riboflavin as a function of pH, which produced an overestimation of the amount released with acid pH.

It was therefore chosen to determine the amount of riboflavin within tablets by emptying the vessel and recovering the tablets.

The analytical results confirmed what was observed visually. Such a test was considered suitable for quantification, by difference, of the release of active substances insoluble, or almost insoluble, in water over time, monitoring their quantity within the tablet with a method of sampling distributed over the eight-hour test time.

It has thus been surprisingly observed that hyaluronic acid can be used as an excipient/component to modulate the release of active ingredients in matrix systems containing non-ionic polymers, such as HPMC.

The same series of tests was carried out for the nicotinamide (Vitamin PP).

EXPERIMENTAL SECTION

A method for the quantitative analysis of Riboflavin

Instrumentation

- HPLC mod. 1220 Infinity LC (Agilent Technologies) equipped with:

  - Zorbax SB-C18 Column ($5\mu$m) 250 mm x 4.6 mm i.d. (Agilent Technologies);
  - Metaguard 2.0 Polaris C18 Ether Precolumn ($3\mu$m) 2 mm x 4.6 i.d. (Agilent Technologies);
  - Variable wavelength UV detector;
  - Computer with ChemStation software ver. B.04.03 (Agilent Technologies) for chromatographic data processing and instrument control.

Chromatographic conditions:

Mobile phase:

Phase A: 50 mM $K_2HPO_4$ solution brought to pH 3.0 by addition of $H_3PO_4$ in ultrapure water.
Phase B: Acetonitrile
Gradient: 0 to 10 min 85% Phase A and 15% Phase B;
Flow rate: 1 mL/min;
Injection volume: $20\mu$L;
Wavelength detector: 282 nm

We proceeded by analyzing the Riboflavin standard at different concentrations, in order to determine the LOD

(1 $\mu$g/mL).

Method for the quantitative analysis of Riboflavin

Instrumentation

HPLC mod. 1220 Infinity LC (Agilent Technologies) equipped with:

Zorbax SB-C18 Column (5$\mu$m) 250 mm x 4.6 mm i.d. (Agilent Technologies);
-Metaguard 2.0 Polaris C18 Ether Precolumn (3$\mu$m) 2 mm x 4.6 i.d. (Agilent Technologies);
Variable wavelength UV detector;
Computer with ChemStation software ver. B.04.03 (Agilent Technologies) for chromatographic data processing and instrument control.

Chromatographic conditions:

Mobile phase:

Phase A: 50 mM $K_2HPO_4$ solution brought to pH 3.0 by addition of $H_3PO_4$ in ultrapure water.
Phase B: Acetonitrile
Gradient: 0 to 10 min 90% Phase A and 10% Phase B;
Flow rate: 0.5 mL/min;
Injection volume: 20$\mu$L;
Wavelength detector: 260 nm

We proceeded by analyzing the Nicotinamide standard at different concentrations, in order to determine the LOD (equal to 1 $\mu$g/mL).

EXAMPLE 2

Description of the formulation of granules and microgranules containing at least one active ingredient according to an innovative calculation method

The solubility in water has proved more useful and convenient (due to easier determination and availability of data) for the purpose of tuning the method. This characteristic was used to build a scale of hydrophilicity-lipophilicity for a large number of sample molecules, chosen from the pharmaceutical and not pharmaceutical active ingredients.
The solubility (S) of a solute is the maximum amount of solute that dissolves in a fixed amount of a particular solvent at a specified temperature.
For the purpose of determining the (requested) hydrophilicity-lipophilicity for an active ingredient, in addition to water solubility, other data were useful as well, such as log Pow, solubility in acid and alkaline solutions or in organic solvents.
The first step is to provide a dosage form or release system of active ingredients for which you want to achieve a given release, as a function of their absorption window, with the matrix system previously described. If so provided, the cores can be coated by a thin film, which may also be enteric.
The second step consists in carrying out a dissolution test, on coated and non coated tablets, in order to verify the behavior of the base system.
In case a change of the delivery of one or more active ingredients is considered useful, the hydrophilicity lipophilicity index for a single dose $I_{p.a.}$ of the active ingredient must be determined according to the following formula, obtained experimentally:

$$I_{p.a.} = \frac{9000\, e^x - 9000\, e^{-x}}{450 e^x - 450 e^{-x} + 10} \cdot \frac{x^{30}}{x^{30} + 0.01}$$

Where $I_{p.a.}$ means the hydrophilicity-lipophilicity index for the active ingredient in single dose.

Coefficient X is equal to the solubility of the active ingredient S (mg/liter) divided by the amount of active ingredient expressed in mg per single dose (mg/dose).

The solubility S of the AP is expressed in mg of substance that is dissolved in 1000 ml of water at the Temperature of 20°C-25°C.

In the case of substances that show a significant difference in solubility between the acid environment and the neutral environment, you will have to make appropriate assessments.

The parameter X is very important, in that it represents the amount of active ingredient that a single dose must carry, since the need of a treatment of the same is strongly influenced by this latter.

The solubility of ionic substances should possibly be verified at the pH of the stomach (about pH = 1).

A substance that is miscible in any ratio with water has an infinite water solubility, in such case considering a substance as the solute and the other as the solvent may be meaningless. Therefore, in this condition use of the above equation, which presents a horizontal asymptote, is not necessarily required. Instead, the value 20 on the scale of hydrophilicity lipophilicity is arbitrarily assigned, while the value 0, or better tending to 0, is attributed to substances considered insoluble in water.

In the case of molecules considered insoluble, the value on the solubility index scale will tend to zero, and therefore, the scale can be considered for convenience between the values 0 -20.

Following this criterion, the substance the Ip.a. index of which was determined will be placed in intimate contact, according to the most useful technique to achieve the purpose, with an adjuvant, for which the index of hydrophilicity-lipophilicity ($I_c$) has been determined in a different way.

For the calculation of $I_c$, the percentage by weight of the hydrophilic portion with respect to the total molecule must be taken into account, according to the following formula:

$$ Ic = \frac{MWi}{MW} \cdot 100 \cdot \frac{1}{5} $$

The formula can be simplified as follows:

$$ Ic = \frac{MWi}{MW} \cdot 20 $$

where MWi is the molecular weight of the hydrophilic portion of the adjuvant and MW is the molecular weight of the entire adjuvant.

The hydrophilicity-lipophilicity scale of adjuvants is also calculated over values ranging from 0 to 20.

Example of calculation, Polyoxyl 40 stearate:

The calculation can be done quickly, considering the constituents of the amphiphilic substance as formed from stearic acid M.W. 248.5 and polyethylene-40 and then by 40 repeating units of about 44 * 40 = 1760 Daltons. The sum of the two hydrophilic and lipophilic portions is then approximately equal to 2008.5 Daltons. By doing so you get an index $I_c$ of 17.5.

In doing so, you must considered that the calculation returns an approximate value, as no information appears therein about the chemical affinity between the active ingredient and adjuvant. It should be added that also in this specific case, the number 40 represents the average value of the hydrophilic portion of polyethylene.

From the method you can understand that calculating with excessive accuracy the number $I_c$ does not make any sense, and, vice versa, that the true composition of the raw material being used is more important (for example, the diester presents a lower amphiphilicity as compared to monoester).

This aspect is apparent when considering complex raw materials, as in the case of lecithins, in which there can be a variation as regards both the fatty acids present in position 1 and 2 on the glycerol molecule, and the alcohol radical (amino, amino acid with alcohol group, sugar) bound to the ortho phosphoric acid, which is in turn bound to the glycerol molecule.

The process conditions include removal of the solvent from the semifinished product.

If the active ingredient is combined with an adjuvant having $I_c$ greater than $I_{p.a.}$ the release increases, all in proportion to the variation between $I_{p.a.}$ and $I_c$.

If the active ingredient is combined with an adjuvant having $I_c$ smaller than $I_{p.a.}$ the release decreases, in a way proportional to the variation between $I_{p.a.}$ and $I_c$.

If an adjuvant with the desired $I_c$ is not available, two adjuvants having different $I_c$S, one higher and one smaller, can be mixed in the right proportions, so that the sum of the two fractions returns that value.

Adjuvants with $I_c$ equal to $I_{p.a.}$ of the substance can be used as diluents.

This adjuvant, or mixture of adjuvants, fall into the category of those substances that are often referred to as wetting agents, surfactants, emulsifiers, diluents.

This system can be used to modify the release of active ingredients over time or to move it to the desired site of action.

The solubilities of some suitable active ingredients are given below:

| Substance | Solubility | S (mg/l) | dose (mg) | X=(mg/l)/ (mg/dose) | $I_{p.a.}$ |
|---|---|---|---|---|---|
| Vitamin A | practically insoluble in water | 0.00001 | 0.1 | 0.0001 | 0.00000 |
| Vitamin E | practically insoluble in water | 0.00001 | 10 | 0.000001 | 0.00000 |
| Riboflavin (form A) | 1g/3-15L ** | 33 | 2 | 16.5 | 20.00000 |
| Riboflavin (form A) | 1 g/3-15L ** | 33 | 40 | 0.825 | 4.69503 |
| Riboflavin (form B) | 80mg/L | 80 | 8 | 10 | 19.99998 |
| L-Methionine | 48 g/l at 293 K | 48000 | 300 | 160 | 20.00000 |
| resveratrol | 0.003g in 100 ml | 30 | 10 | 3 | 19.97784 |
| Ubidecarenone | practically insoluble in water | 0.00001 | 10 | 0.000001 | 0.00000 |
| Dimethyl sulfone or methyl sulfonyl methane | 150 g/L at 25°C | 150000 | 500 | 300 | 20.00000 |
| tyrosine | 25°C | 453 | 45.3 | 10 | 19.99998 |
| tyrosine | 25°C | 453 | 453.0 | 1 | 19.61651 |
| tyrosine | 25°C | 453 | 498.3 | 0.909090909 | 16.84853 |
| tyrosine | 25°C | 453 | 543.6 | 0.833333333 | 5.85835 |
| tyrosine | 25°C | 453 | 588.9 | 0.769230769 | 0.72578 |
| tyrosine | 25°C | 453 | 634.2 | 0.714285714 | 0.08114 |
| * Variations in solubility are due to different crystal forms | | | | | |

Examples of suitable lecithins:

lecithin dissolved in ethyl alcohol, which also enables storage at room temperature for 12 months, obtained from vegetable raw materials (Glycine max L.) in which the most representative phospholipid component is the following:

70-85% 3-sn-phosphatidylcholine
3-10% 3-sn-Lysophosphatidylcholine
3-10% Phosphatidylethanolamine
3-10% Acid fasfatidico

The ethyl alcohol was then removed.

Lecithin powder rich in phosphatidylcholine (not less than 90%) with lysophosphatidylcholine (not more than 6%).

Lecithin powder rich in phosphatidylcholine (not less than 94-102%) with lysophosphatidylcholine (not more than 4%).

Lecithin powder rich in phosphatidylcholine (not less than 85%) with lysophosphatidylcholine (not more than 6%, phosphatidic acid not more than 7%, phosphatidylethanolamine not more than 7%, non-polar lipids not more than 4,0%).

Example 3

Tests of mixing/granulation of the active ingredient with adjuvants with different hydrophilic-lipophilic index

Granulates containing riboflavin were produced using adjuvants with different hydrophilicity-lipophilicity index $I_c$.
According to the Merck Index, the solubility of the riboflavin is between 1g in 3-15 liters, depending on the crystalline form being used.
The solubility of riboflavin was then deduced experimentally. For this we obtained a solubility at 20°C in the order of 80 mg/liter.
Water-soluble active ingredient: Riboflavin

TEST PS1

| | |
|---|---|
| Riboflavin | 7.5 % |
| propylene glycol monolaurate (type I) EP/NF Ic = 4 | 30% |
| Sucrose palmitate (mono-di and triesters of sucrose with palmitic acid) (Ic = 15) | 46% |
| Silicon Dioxide 1 | 16.50% |
| Overall $I_c$ = 8. | |

Method of preparation: Introduce the sucrose ester and propylene glycol monolaurate in a fast homogenizer, melt the mass and add the riboflavin, mix to homogeneity. Add the silica and start cooling. After reaching the solidification, reduce the granulate to the desired size by an oscillating arm granulator equipped with a special network.

TEST PS2

| | |
|---|---|
| Riboflavin | 7,50% |
| Propylene glycol monolaurate (type I) EP/NF ($I_c$ = 4) | 30% |
| Glyceryl dipalmitostearate E471/GRAS ($I_c$ = 2) | 46% |
| Silicon Dioxide | 16.50% |

$I_c$ = 2.83
Method of preparation: Introduce the propylene glycol monoolaurate and glycerol dipalmitostearate, in this order, in a fast mechanical homogenizer, melt the mass and add the riboflavin, mix to homogeneity. Add the silica and start cooling. Pass through a sieve by an oscillating arms granulator.
The recommended daily intake of riboflavin is 1.4 mg/day, and indicates the amount of vitamin B2 that a person should take daily to meet the personal minimum need of that vitamin. These particulars relating to the needs of nutritional factors may vary from country to country, or even over time as a result of revaluation of Scientific Committees. However, you can consider realistic an administration of riboflavin between 0.3 mg/day to a maximum of 2 mg. Therefore, there are no solubility problems with a dose of 2 mg, and in a complex system, containing insoluble vitamins and soluble vitamins, erosion of the matrix of hyaluronic acid/non-ionic polymer should be such as to produce a progressive erosion of the core so as to allow the release of insoluble vitamins. However it may occur that, upon setting the correct amount of the two polymers, the delivery in time of one or more soluble vitamins must be reduced. The examples previously described for the riboflavin, show how the delivery of this ingredient can be reduced by applying the calculation method with a dose of 2 mg, corresponding to a $I_{p.a}$. ~/=20.

EXAMPLE 4

Fat-soluble active ingredient: Ubidecarenone

The ubidecarenone or coenzyme Q-10 is a molecule of the group of fat-soluble ubiquinones, benzoquinones involved in electron transportation in mitochondria and the cell membrane. The ubidecarenone is nowadays proposed as a dietary supplement in combination with other antioxidants such as vitamin C, vitamin E, beta-carotene, selenium and zinc, to counteract the ageing of the organism.

The usual dose for adults is 10-20 mg three times a day or 50 mg in one daily administration, after meals. The ubidecarenone is practically insoluble in water, as indeed are Vitamin A and Vitamin E. Accordingly, given the poor solubility, the only way to facilitate the release from the dosage form is to bring it into intimate contact with adjuvants particularly hydrophilic and therefore with high $I_c$.

TEST PS3

| | |
|---|---|
| Ubidecarenone | 5% |
| Lauroyl macrogol-32 glycerides EP ($I_c = 14$) | 68% |
| Magnesium Carbonate | 27% |

$I_c = 2.83$

Method of preparation: Introduce the ubidecarenone and magnesium carbonate, in this order, in a Viani fast granulator, equipped with a chopper and four-way mixing system. Melt the Lauroyl macrogol-32 glycerides EP in a container of appropriate size. Add the Lauroyl macrogol-32 glycerides EP to the powder mixture, knead the product to obtain a homogeneous mixture. Discharge and pass through a sieve by an oscillating arms granulator, vibrating screen or equivalent.

In parallel, the ubidecarenone is treated with an adjuvant with low $I_c$. In this way we intend to demonstrate the relationship between low $I_c$.

TEST PS4

Ubidecarenone 5%

Polyglycerol 3 dioleate (liquid) ($I_c = 6$) 68%

Magnesium Carbonate 27%

$I_c = 6$

EXAMPLE 5

Release system in the form of uncoated tablet

| | |
|---|---|
| Dicalcium phosphate | 300 mg |
| Microcrystalline cellulose | 300 mg |
| Hyaluronic acid MW < 1000 Da | 7 mg |
| hydroxypropyl methylcellulose | 47 mg |
| Magnesium stearate | 7 mg |
| Silicon dioxide | 3.5 mg |
| Granular riboflavin (riboflavin, propylene glycol monolaurate sucrose palmitate, silicon dioxide. Title 7.5%) | 18,66 mg |
| 50% powdered tocopheryl acetate | 36 mg |

EXAMPLE 6

Release system in the form of a tablet provided with an enteric layer

| | |
|---|---|
| Granular L-Methionine (85% methionine) | 352.94 mg |
| N-(3-aminopropyl)-tetramethylene trihydrochloride | 0.55 mg |
| Zeaxanthin vegetarian badlets (zeaxanthin 5%) | 44 mg |

(continued)

| | |
|---|---|
| Rutin | 2.99 mg |
| Calcium d-pantothenate | 9.90 mg |
| d-Biotin | 0.055 mg |
| Ascorbic acid (97%) | 101.5 mg |
| vitamin E acetate (50%) | 36.0 mg |
| Pyridoxine hydrochloride | 2.67 mg |
| Zinc bisglycinate | 26.60 mg |
| Copper bisglycinate | 4 mg |
| Folic Acid | 0.22 mg |
| Vitis vinifera leaves and e.d. seeds | 60 mg |
| Olive leaf extract | 10 mg |
| Selenium yeast | 30 mg |
| Dicalcium phosphate | 24 mg |
| Hydroxypropyl methylcellulose | 10 mg |
| Hyaluronic acid MW < 1000 Da | 10 mg |
| Microcrystalline cellulose | 65 mg |

Enteric coating based on Sodium Alginate, Polyethylene glycol, Potassium Sorbate, Talc 13.4 mg

**Claims**

1. A solid system in the form of a tablet or capsule for the modified release and oral administration of one or more active ingredients, including a polymeric matrix comprising an ionic polymer which is hyaluronic acid in the form of free acid with a weighted-average molecular weight ranging from 100 to 100,000 Daltons (Da) measured with SEC-MALLS technique, a non-ionic polymer which jellifies in contact with a liquid, and an active ingredient dispersed in the polymeric matrix.

2. A solid system for the modified release in accordance with claim 1, wherein the hyaluronic acid in the form of free acid has a weighted-average molecular weight ranging from 500 to 50,000 Daltons (Da).

3. A solid system for the modified release in accordance with claim 1 or 2, wherein the non-ionic polymer is a cellulose based polymer, a cellulose derivative, or an ethylene oxide polyether and/or mixtures thereof.

4. A solid system for the modified release in accordance with any of claims 1-3, wherein said active ingredients are in the form of microgranules or microcapsules dispersed in the polymer matrix.

5. A solid system for the modified release in accordance with claim 4, wherein the microgranules comprise an active ingredient and an amphiphilic adjuvant.

6. A solid system for the modified release in accordance with any of claims 1-5, wherein the active ingredients comprise vitamins, aminoacids, minerals, pharmacologically active ingredients, plant extracts, phytocompounds, probiotic microorganisms and mixtures thereof.

7. A solid system for modified release in accordance with any of claims 1-6, further comprising an enteric coating.

8. A solid system for modified release in accordance with claim 7, wherein the enteric coating comprises cellulose derivatives, preferably selected from cellulose acetate phtalate, cellulose acetate propionate, acrylic or methacrylic acid derivatives, shellac, modified starched, alginates, propolis and mixtures thereof.

9. A solid system in the form of a tablet or capsule for oral administration comprising a polymeric matrix comprising an ionic polymer which is hyaluronic acid in the form of free acid with a weighted-average molecular weight ranging from 100 to 100,000 Daltons (Da) measured with SEC-MALLS technique, a non-ionic polymer which jellifies in contact with a liquid, for use in modulating the release of one or more active ingredients from the solid system.

10. The solid system for use in accordance with claim 9, wherein said non-ionic polymer is selected from hydroxypropyl methylcellulose, methylcellulose, hydroxypropyl cellulose, hydroxyethyl cellulose, carboxyethyl cellulose, or an ethylene oxide polyether and mixtures thereof.

11. The solid system for use in accordance with claim 9 or 10, wherein the solid system is in form of a tablet and the active ingredient is contained in microgranules or microcapsules dispersed in the polymeric matrix.

## Patentansprüche

1. Feststoff-System in Form einer Tablette oder Kapsel zur angepassten Abgabe und oralen Verabreichung von einem oder mehreren Wirkstoffen mit einer Polymermatrix, aufweisend ein ionisches Polymer, das Hyaluronsäure in Form von freier Säure ist, mit einem gewichteten Durchschnitts-Molekulargewicht zwischen 100 und 100.000 Dalton (Da), gemessen mit der SEC-MALLS-Technik, ein nicht-ionisches Polymer, das bei Kontakt mit Flüssigkeit geliert, und einen Wirkstoff, der in der Polymermatrix dispergiert ist.

2. Feststoff-System zur angepassten Abgabe gemäß Anspruch 1, wobei die Hyaluronsäure in Form von freier Säure ein gewichtetes Durchschnitts-Molekulargewicht zwischen 500 und 50.000 Dalton (Da) hat.

3. Feststoff-System zur angepassten Abgabe gemäß Anspruch 1 oder 2, wobei das nicht-ionische Polymer ein zellulose-basiertes Polymer, ein Zellulose-Derivat, oder ein Ethylenoxid-Polyether und / oder Mischungen davon ist.

4. Feststoff-System zur angepassten Abgabe gemäß einem der Ansprüche 1 bis 3, wobei die Wirkstoffe in Form von Mikrokörnchen oder Mikrokapseln, dispergiert in der Polymermatrix, vorliegen.

5. Feststoff-System zur angepassten Abgabe gemäß Anspruch 4, wobei die Mikrokörnchen einen Wirkstoff und einen amphiphilen Hilfsstoff aufweisen.

6. Feststoff-System zur angepassten Abgabe gemäß einem der Ansprüche 1 bis 5, wobei die Wirkstoffe Vitamine, Aminosäuren, Mineralien, pharmakologische Wirkstoffe, Pflanzenextrakte, Phytokomplexe, probiotische Mikroorganismen und Mischungen davon aufweisen.

7. Feststoff-System zur angepassten Abgabe gemäß einem der Ansprüche 1 bis 6, weiter aufweisend eine magensaftresistente Beschichtung.

8. Feststoff-System zur angepassten Abgabe gemäß Anspruch 7, wobei die magensaftresistente Beschichtung Zellulose-Derivate aufweist, vorzugsweise ausgewählt aus Zellulose-Azetat-Phtalat, Zellulose-Azetat-Propionat, Acrylsäure-Derivaten oder Methacrylsäure-Derivaten, Schellack, modifizierten Stärken, Alginaten, Propolis und Mischungen davon.

9. Feststoff-System in Form einer Tablette oder Kapsel zur oralen Verabreichung aufweisend eine Polymermatrix, aufweisend ein ionisches Polymer, das Hyaluronsäure in Form von freier Säure ist, mit einem gewichteten Durchschnitts-Molekulargewicht zwischen 100 und 100.000 Dalton (Da), gemessen mit der SEC-MALLS-Technik, und ein nicht-ionisches Polymer, das bei Kontakt mit Flüssigkeit geliert, zur Verwendung bei modulierender Abgabe von einem oder mehreren Wirkstoffen aus dem Feststoff-System.

10. Das Feststoff-System zur Anwendung gemäß Anspruch 9, wobei das nicht-ionische Polymer ausgewählt ist aus Hydroxypropylmethylzellulose, Methylzellulose, Hydroxypropylzellulose, Hydroxyethylzellulose, Carboxyethylzellulose, oder einem Ethylenoxid-Polyether und Mischungen davon.

11. Das Feststoff-System zur Anwendung gemäß Anspruch 9 oder 10, wobei das Feststoff-System in Form einer Tablette vorliegt und der Wirkstoff in Mikrokörnchen oder Mikrokapseln, dispergiert in der Polymermatrix, enthalten ist.

**Revendications**

1.  Système solide sous la forme d'un comprimé ou d'une capsule pour la libération modifiée et l'administration orale d'un ou de plusieurs ingrédients actifs, incluant une matrice polymère comprenant un polymère ionique qui est un acide hyaluronique sous forme d'acide libre ayant un poids moléculaire moyen en poids allant de 100 à 100 000 Daltons (Da) mesuré avec la technique SEC-MALLS, un polymère non ionique qui se gélifie au contact d'un liquide, et un ingrédient actif dispersé dans la matrice polymère.

2.  Système solide pour la libération modifiée selon la revendication 1, dans lequel l'acide hyaluronique sous forme d'acide libre présente un poids moléculaire moyen en poids allant de 500 à 50 000 Daltons (Da).

3.  Système solide pour la libération modifiée selon la revendication 1 ou 2, dans lequel le polymère non ionique est un polymère à base de cellulose, un dérivé de cellulose, ou un oxyde d'éthylène polyéther et/ou des mélanges de ceux-ci.

4.  Système solide pour la libération modifiée selon l'une quelconque des revendications 1 à 3, dans lequel lesdits ingrédients actifs sont sous la forme de microgranules ou de microcapsules dispersés dans la matrice polymère.

5.  Système solide pour la libération modifiée selon la revendication 4, dans lequel les microgranules comprennent un ingrédient actif et un adjuvant amphiphile.

6.  Système solide pour la libération modifiée selon l'une quelconque des revendications 1 à 5, dans lequel les ingrédients actifs comprennent des vitamines, des acides aminés, des minéraux, des ingrédients pharmacologiquement actifs, des extraits de plante, des phytocomposés, des micro-organismes probiotiques et des mélanges de ceux-ci.

7.  Système solide pour la libération modifiée selon l'une quelconque des revendications 1 à 6, comprenant en outre un enrobage entérique.

8.  Système solide pour la libération modifiée selon la revendication 7, dans lequel l'enrobage entérique comprend des dérivés de cellulose, de préférence sélectionnés parmi un phtalate d'acétate de cellulose, un propionate d'acétate de cellulose, des dérivés d'acide acrylique ou méthacrylique, une gomme laque, un amidon modifié, des alginates, de la propolis et des mélanges de ceux-ci.

9.  Système solide sous la forme d'un comprimé ou d'une capsule pour administration orale comprenant une matrice polymère comprenant un polymère ionique qui est un acide hyaluronique sous forme d'acide libre ayant un poids moléculaire moyen en poids allant de 100 à 100 000 Daltons (Da) mesuré avec la technique SEC-MALLS, un polymère non ionique qui se gélifie au contact d'un liquide, pour une utilisation dans la modulation de la libération d'un ou de plusieurs ingrédients actifs à partir du système solide.

10. Système solide pour une utilisation selon la revendication 9, dans lequel ledit polymère non ionique est sélectionné parmi une hydroxypropyl méthylcellulose, une méthylcellulose, une hydroxypropyl cellulose, une hydroxyéthyl cellulose, une carboxyéthyl cellulose, ou un oxyde d'éthylène polyéther et des mélanges de ceux-ci.

11. Système solide pour une utilisation selon la revendication 9 ou 10, dans lequel le système solide est sous la forme d'un comprimé et l'ingrédient actif est contenu dans des microgranules ou des microcapsules dispersés dans la matrice polymère.

**REFERENCES CITED IN THE DESCRIPTION**

**Patent documents cited in the description**

- WO 2006087392 A